# EUROPEAN PATENT SPECIFICATION

(11) **EP 4 257 098 B1**
(45) Date of publication and mention of the grant of the patent: **28.10.2026**
(21) Application number: 23194042.0
(22) Date of filing: 11.03.2020
(51) Int. Cl.: A61F 5/443, A61F 5/445, A61F 5/44, A61F 5/441, A61F 5/451

(54) **BODY WASTE COLLECTING SYSTEM**
SYSTEM ZUM SAMMELN VON KÖRPERABFÄLLEN
SYSTÈME DE COLLECTE DE DÉCHETS CORPORELS

(30) Priority: 11.03.2019 DK PA201900307; 05.09.2019 DK PA201901049
(43) Date of publication of application: 11.10.2023
(62) Document number(s) of the earlier application(s) in accordance with Art. 76 EPC: 20715689.4 / 3 937 862
(73) Proprietor: Furine ApS, 3320 Skævinge (DK)
(72) Inventor: Nielsen, Brian Thomsen, 3330 Gørløse (DK)
(74) Representative: Münzer, Marc Eric

(56) References cited:
- WO-A1-2005/102229
- WO-A1-2018/050856
- WO-A1-2018/188706
- GB-A- 1 550 960
- US-A1- 2007 219 514
- US-A1- 2012 041 404
- US-A1- 2012 302 981
- US-A1- 2012 323 193

## Description

### Field of the invention

The invention presented in this specification is related to a faecal collecting system.

The invention relates to a body waste collecting system arranged to be attached to the perianal skin of the user as defined in the appended claims. Embodiments, examples or aspects in the following disclosure which do not fall under the scope of the claims are presented for illustration purposes only and do not form part of the invention.

### Description of related art for the first embodiment

Many ostomy applications are disclosed in the prior art. Some are arranged as 1-piece collecting bags attached directly to the peri-stomal skin and some are arranged as 2-piece systems with a wafer attached directly to the peri-stomal skin and with a coupling for detachably attaching a bag in such a manner that the bag may be changed more frequently than the wafer. Most systems are attached via adhesive means.

It is known, that output from the ostomy may migrate under the adhesive causing the adhesive to separate from the skin. Problems with such separation are leakages and skin irritation (stomal output may be extremely potent in skin degradation). This is often solved in the prior art by use of liquid absorbing adhesives such as Hydrocolloid. This increases the time before the applied adhesive separates from the skin and reduces the wetting of the skin. However the problem remains.

When inventing the skin attaching portion of an ostomy application, some have tried to mix adhesives and thereby obtain zones with different adhesive properties. Such properties include absorption, adhesive power, flexibility and permeability of the wafer. A wafer construction with zones having different properties may also be achieved by different supporting layers attached to the skin contacting adhesive.

Examples of such ostomy applications comprising an at least 2-zoned adhesive wafer construction with different properties include EP2654633B1, US2014323941A, US2014114265A, US2013274696A and WO 05102229A1.

EP2654633B1 describes a wafer construction for attachment to the peri-stomal skin and being capable of attaching a collecting bag to its outer surface. The wafer comprises a central zone, an intermediate zone and an edge zone. The central zone and edge zone comprise a pressure sensitive adhesive with high adhesive power while the central zone comprises a soft adhesive with lower adhesion power. The soft adhesive has high moist wapor transmission rate (p. 4, line 14-16). The wafer may comprise an impervious backing layer. Furthermore, the wafer may comprise an absorbent layer behind the soft adhesive (p. 4, line 29-32 and figures).

WO2012 083964A1 describes a wafer construction for attachment to the peri-stomal skin. The wafer comprises a central zone and an edge zone. The edge zone provides the majority of the adhesive power and may be moist vapour permeable. The central zone comprises a cushioning layer and may be moist vapour impermeable. The cushioning layer may be a hydrocolloid adhesive layer. The construction comprises a collecting bag to be attached to the wafer either directly (one piece system) via a flange system for detachably attaching the bag to the wafer if designing a two piece system (section 10; p.4 line 26 to p.5 line 2).

Most ostomy collecting systems comprise a skin-attaching wafer, said wafer comprising a skin adhesive on the inside. When analysing the in-use-situation of ostomy bag systems and when viewing the radially extending skin attaching portion from the ostomy, there are two areas. A first central stomal-adjacent-area where the outside of the central portion of the skin-attaching wafer is in fluid communication with the interior of the collecting bag. Also a second encircling area radially more distant from the ostomy, where the outside of the skin-attaching wafer is not in fluid communication with the interior of the collecting bag.

In the interior of the collecting bag, there is mal-odour and 100 % humidity. Even though the prior art may solve flexibility, absorption of liquid and to a certain extent moisture evaporation; when focussing on moisture evaporation from the peri-stomal skin, vapour transportation and water condensation the prior art suffers from a significant drawback. They bring either:
a) via a permeable layer the central stomal-adjacent-area of the skin in vapour communication with the interior of the bag; or
b) they arrange the central stomal-adjacent-area of the wafer to be substantially moisture impermeable.

EP2654633B1 is only capable of handling a certain amount of liquid due to the encapsulated absorbent layer and the bag construction and does not sufficiently handle the combination of strong adherence, skin protection and high skin evaporation. Particularly, the skin in the central stomal-adjacent-area will not be able to expose moisture, when the absorbent layer is saturated.

WO2012 083964A1 is only capable of handling moist vapour evaporation from the portion of the skin extending radially outward from the collecting bag or coupling arrangement.

There remains a need for a wafer construction and ostomy device, which allows skin respiration in the central stomal-adjacent-area in such a manner that the skin adhesive remains sufficiently attached to the skin.

The first embodiment is in particular useful on users that are relatively active or have increased temperature and therefore have increased skin perspiration.

### Summary of the first embodiment

In a first aspect, the present disclosure relates to a wafer construction (200) for applying a collecting system (100) around an ostomy, the wafer construction comprising:
a. a central through-going aperture (201) for bringing the ostomy (001) in fluid communication with a collecting reservoir (300),
b. an inner radially extending layer (220) for attaching the wafer to the peri-stomal skin (002); said inner layer (220) comprising:
   i. a skin contacting surface (221);
   ii. a moist vapour permeable layer (224);
   iii. an outer surface (226);
c. an outer radially extending layer (230); said outer layer comprising:
   i. a substantially moist vapour impermeable film (232);
   ii. an outer surface (236);
   iii. an inner surface (238);
   iv. a bag element (234) for engaging the wafer (200) to a collecting reservoir (300);
d. a central stoma-adjacent-area (240) comprising a first area of both the inner layer (220) and the outer layer (230); said central stoma-adjacent-area defined radially as the area of the wafer construction (200) closer to the central through-going aperture (201) than the bag element (234) of the outer layer;
e. an encircling area (250) comprising a second area of at least the inner layer (220);
f. at least one radial space (270) in fluid communication with the atmosphere (500); said radial space (270) being located between the outer surface of the inner layer (226) and the inner surface of the outer layer (238); and
g. at least one attachment point (260); said attachment point attaching the outer layer (230) to the inner layer (220);
wherein at least a portion of the radial space (270) is located in the central stoma-adjacent-area (240).

In a second aspect, the present disclosure relates to a collecting system (100) application on the peri-stomal skin, said system comprising:
a. a wafer construction (200) according to the first aspect of the present invention; and
b. a collecting reservoir (300) for collecting the stomal output.

In a third aspect, the present disclosure relates to a method of providing a wafer construction (200) or collecting system (100) according to the first or second aspect of the present disclosure, said method comprising the steps of:
a. providing a wafer construction (200) according to the first aspect of the present invention; and
b. packing the wafer construction.

### Description of related art for the second embodiment

Body waste collecting systems are known in the art. For example, reference is made to WO 2018/050856 A1, WO 2018/050856 and WO 2019/179586. Body waste collecting systems need to be attached to the user's body. Certain systems use an adhesive to attach an attachment member to the user's body. WO 2018/050856 A1 discloses body waste collecting system arranged to be attached to the peri-anal skin of the user.

If the adhesive on the attachment member detaches from the user's body, there is a risk that body fluids and or odours leak from the user. It is desired to reduce this risk as much as possible. Some prior art systems could be said to have a greater risk of detachment than others due to the mechanical arrangement of the attachment member.

### Summary of the second embodiment

It is therefore a first aspect of the second embodiment to provide a body waste collecting system which has an attachment member with a more secure connection to the user's body.

A body waste collecting system in the form of a perianal faecal collecting system similar to the kind described in this specification is also described in more detail in WO 2018/050856 A1, WO 2018/050856 and WO 2019/179586. Many of the features disclosed in said applications are relevant for the system disclosed in this specification.

A body waste collecting system according to the second embodiment comprises
a) an attachment member arranged to be attached to the user, said attachment member comprising an opening which is arranged to be in fluid communication with the anus of the user or with a stoma of the user and an attachment flange which is arranged to encircle said opening and which is arranged to be attached to the perianal or peristomal skin of the user such that a fluid tight seal is established between the attachment flange and the perianal or peristomal skin of the user around the anus or stoma respectively, said attachment flange being provided with a skin friendly adhesive on a skin contacting surface of said attachment flange, and b) a collection bag connected to the attachment member, said collection bag being suitable for collecting the body waste output. In one embodiment, the body waste collecting system further comprises an elongated tubular section or elongated hollow conduit having a first end and a second end, said first end being connected to said attachment member and being in fluid communication with the opening of said attachment member and said second end being in fluid communication with said collection bag. In one embodiment, the elongated hollow conduit or tubular section is at least 10cm, at least 20cm or at least 30cm long.

It should be noted that the body waste collecting system shown in the drawings and in accordance to the invention is designed to collect faecal waste from the anus of a user

However, the body waste collecting system could also be used to collect faecal waste coming from a stoma of a user or urine coming from a urostomy. Instead of mounting the attachment member to the perianal skin around the anus, the attachment flange could be adhered to the peristomal skin around the stoma of a user. As the stoma typically protrudes from the skin, the stoma can be arranged to fit inside the opening of the attachment member. An ostomy wax or ostomy ring could be applied between the stoma and the attachment member to further seal the connection between the stoma and the attachment member. This type of body waste collecting system could be attached to colostomies, ileostomies and urostomies and collect faecal matter or urinary matter.

In one embodiment the collecting bag is detachably attached to the outlet of the tubular section or conduit, for example via a connecting mechanism.

In one embodiment, the attachment flange of the attachment member is arranged as a two layer structure, as is described with respect to the first embodiment related to an ostomy wafer. By providing an inner (with respect to the user) permeable film and an outer impermeable film separated by an air permeable gap, the user's skin is able to breathe through the attachment member without increasing the risk of odour leakage through the attachment member. In one embodiment, the air permeable gap is a space between the two layers. In one embodiment, the air permeable gap is filled with a material which is air permeable, as described with regards to the first embodiment above.

In one embodiment, the attachment flange could be provided with an inner flange portion arranged closest to the opening in the attachment member and an outer flange portion arranged further away from the opening in the attachment member than the inner flange portion and a tubular section extending away from the attachment flange, said tubular section being in fluid communication with the opening in the attachment flange and being attached to the attachment flange at a location between the inner and outer flange portions. In this way, the attachment member will have a T-shaped cross section on both sides of the tubular member when taking a cross section through the attachment member on a plane which is perpendicular to the attachment flange.

During use of such a faecal collecting system, the user may unintentionally pull in the tube, and thereby pull on the attachment flange. Due to the T-shaped cross section, when force is applied to the tubular section, the force will be applied to the middle of the attachment flange and not to one side of the attachment flange as is the case in the prior art solutions. In this way, peeling of the attachment flange away from the skin is prevented.

In one embodiment of an attachment member having a tubular portion attached between an inner and outer portion of the attachment flange, the attachment flange comprises an annular groove between the inner and outer flange portions. In one embodiment, said groove is filled or is able to be filled with an adhesive.

It may furthermore be beneficial if the adhesive in the groove of the attachment member is sufficiently elastic to distribute such pull force over the entire attachment area.

In one embodiment the adhesive in the groove is soft and elastic. In one embodiment the adhesive in the groove has an elongation at break of from 150 to 4000 % or from 250 to 2000 %.

In one embodiment, the attachment flange comprises an annular flange portion on the inside and/or outside of the annular groove, said annular flange(s) being provided with a skin friendly adhesive.

In one embodiment, said annular flange portion(s) is/are provided with through going openings to allow skin arranged underneath the annular flange portion(s) to breathe. In one embodiment, the through going openings in the annular flange portions could be covered by a thin permeable film which is covered by a skin friendly adhesive.

In one embodiment, the attachment member comprises elongated chimneys circularly arrayed in the annular groove and extending perpendicularly to the plane of the groove, said chimneys connecting the user's skin to the outer surface of the attachment flange. In this way, air can flow through the chimneys from the user's skin to the outside environment. In one embodiment, the chimneys are surrounded by adhesive.

It should be noted that the embodiments shown in the figures show an attachment flange with a roughly circular outer periphery. However within the scope of the current invention, the outer periphery of the attachment flange could take other forms and does not have to be circular.

### Description related to the third embodiment

Tracheostomy tube assemblies are well known in the art. They are usually held in place with a neck strap which goes around the user's neck. However the neck strap is annoying for users.

### Summary of the third embodiment

It is therefore a first aspect of the third embodiment to provide a tracheostomy tube assembly which is easier to use for the user as well as being less annoying for the user.

This aspect is provided at least in part by a tracheostomy tube assembly comprising an internal tubular portion which is arranged to be inserted into the trachea of the user via a tracheostomy, an external tubular portion which extends outside the tracheostomy and an attachment flange connected to the external tubular portion, **characterized** in that the attachment flange comprises a skin friendly adhesive on a skin facing surface of the attachment flange. The skin facing surface of the attachment flange is also the surface of the attachment flange which faces the internal tubular portion.

In one embodiment, the attachment flange comprises two flange portions, each extending outwardly from opposing sides of the external tubular portion. In one embodiment, the attachment flange comprises a flange which extends radially outwardly from the external tubular portion around the entire periphery of the external tubular portion. In one embodiment, the adhesive is applied in an annular shape around the periphery of the external tubular portion to provide a seal between the tracheostomy and the outside environment.

In one embodiment, the attachment flange is detachably connectable to the external tubular portion. In one embodiment, the tracheostomy tube assembly comprises a fastening member, said fastening member being connected between the external tubular portion and the attachment flange. In one embodiment, the fastening member is detachably connected to the attachment flange and/or the external tubular portion.

In one embodiment, the attachment flange is integrally connected to the external tubular portion.

In one embodiment, the tracheostomy tube assembly comprises an absorbing material placed between the tracheostomy and the attachment flange. Such an absorbing material can absorb fluids leaking out of the tracheostomy. In one embodiment, the absorbing material is held in place by the fastening member. In one embodiment, the absorbing material can be echanged by detaching the fastening member from the attachment flange and/or from the external tubular portion.

In one embodiment, the flange comprises a construction similar to the ostomy wafer described above, with a permeable layer on the skin facing side of the flange and an impermeable layer on the outer side.

In one embodiment, the attachment flange is provided with through going openings to ventilate the skin around the tracheostomy. In one embodiment, the through going openings in the attachment flange are covered by a thin permeable film covered by an adhesive.

In one embodiment, the adhesive is a permeable adhesive.

In one embodiment, the attachment flange is made from a permeable material.

In one embodiment, the adhesive on the skin facing surface of the attachment flange is applied to the skin facing surface of the attachment flange in a pattern, so that at least 25%, at least 35% or at least 50% of the skin facing surface of the attachment flange is free of adhesive.

In one embodiment, the skin facing surface of the attachment flange is provided with an annular groove arranged around the periphery of the external tubular section, said annular groove being filled, or being arranged to be filled, with an adhesive.

A second aspect of the third embodiment is a method of applying a tracheostomy tube to a user. The method comprises the steps of inserting an internal tubular member of a tracheostomy tube into a trachea of a user via a tracheostomy in the user, attaching an attachment flange connected to an external tubular portion of the tracheostomy tube to the skin of the user around the tracheostomy via an adhesive applied on a skin facing surface of the attachment flange.

In one embodiment, prior to inserting the internal tubular member into the user, an adhesive is applied to the skin facing surface of the attachment flange.

A third aspect of the third embodiment relates to a tracheostomy kit comprising a tracheostomy tube and a fastening member, said fastening member comprising a skin adhesive system, a tube fastening portion and a reservoir for moisture handling, wherein the tracheostomy tube is fastened to the user via the fastening member.

It should be noted that the features of the second and third embodiments have been described as a number of separate embodiments. It will be clear to the person skilled in the art that these features can be combined in different ways. Claims directed to these features will therefore be provided with multiple claim dependencies as is known to the person skilled in the art of claim drafting.

### Brief description of the drawings

In the following, embodiments of the disclosure will be described in greater detail with reference to embodiments shown by the enclosed figures. It should be emphasized that the embodiments shown are used for example purposes only and should not be used to limit the scope of the invention.
Figures 1a to 1c schematically illustrate different views of an example of a wafer construction (200) and a collecting system (100) according to one embodiment disclosed herein.
Figure 2 is a cross sectional view of an example of a wafer construction (200) and a collecting system (100) according to the one embodiment disclosed herein prior to application.
Figure 3 is a close up of a cross sectional view of the wafer (200) and system (100) from figure 1 when worn by a patient.
Figures 4 to 5 illustrate schematic top views of different patterns of attachment points in examples of wafer constructions (200) and a collecting system (100) according to the one embodiment disclosed herein.
Figure 6 shows a schematic overview of a body waste collecting system according to the second embodiment**.**
Figure 7 shows a schematic side view of an embodiment of an attachment member of a body waste collecting system according to the second embodiment.
Figure 8 shows a schematic top view of the attachment member of figure 7.
Figures 9-11 show respectively a cross sectional view, a top perspective view and a bottom perspective view of an embodiment of an attachment member according to the second embodiment.
Figures 12-14 show respectively a cross sectional view, a top perspective view and a bottom perspective view of an embodiment of an attachment member according to the second embodiment.
Figures 15-17 show respectively a cross sectional view, a top perspective view and a bottom perspective view of an embodiment of an attachment member according to the second embodiment.
Figure 18 shows a schematic cross sectional view of a user with a tracheostomy tube assembly according to the third embodiment inserted into the trachea of the user.
Figure 19 shows a schematic side view of an embodiment of a tracheostomy tube assembly according to the third embodiment.
Figure 20 shows a schematic cross section view of another embodiment of a tracheostomy tube assembly according to the third embodiment.

### Detailed description of the embodiments

The first aspect of the first embodiment disclosed herein relates to a wafer construction (200) for applying a collecting system (100) around an ostomy, the wafer construction comprising:
a. a central through-going aperture (201) for bringing the ostomy (001) in fluid communication with a collecting reservoir (300),
b. an inner radially extending layer (220) for attaching the wafer to the peri-stomal skin (002); said inner layer (220) comprising:
   a. a skin contacting surface (221);
   b. a moist vapour permeable layer (224);
   c. an outer surface (226);
c. an outer radially extending layer (230); said outer layer comprising:
   a. a substantially moist vapour impermeable film (232);
   b. an outer surface (236);
   c. an inner surface (238);
   d. a bag element (234) for engaging the wafer (200) to a collecting reservoir (300);
d. a central stoma-adjacent-area (240) comprising a first area of both the inner layer (220) and the outer layer (230); said central stoma-adjacent-area defined radially as the area of the wafer construction (200) closer to the central through-going aperture (201) than the bag element (234) of the outer layer;
e. an encircling area (250) comprising a second area of at least the inner layer (220);
f. at least one radial space (270) in fluid communication with the atmosphere (500); said radial space (270) being located between the outer surface of the inner layer (226) and the inner surface of the outer layer (238); and
g. at least one attachment point (260); said attachment point attaching the outer layer (230) to the inner layer (220);
wherein at least a portion of the radial space (270) is located in the central stoma-adjacent-area (240).

In the context of the present invention the term "permeability" shall be understood as Moist Vapour Transmission Rate (MVTR) and are measured according to ASTM test method F1249 (at 38°C, 100% RH). The term "substantially moist vapour impermeable" shall be understood as a MVTR below 400 g/m2/24h. The term "moist vapour permeable" shall be understood as a MVTR of at least 500 g/m2/24h.

In one embodiment, the inner radially extending layer (220) has a permeability of at least 600 g/m2/24h. In one embodiment, the inner radially extending layer has permeability of at least 750 g/m2/24h. In one embodiment, the inner radially extending layer has permeability of at least 900 g/m2/24h. Many technologies known in the art can be used to create a permeable layer. These include, but are not limited to breathable films, non-wovens and micro perforated films (when micro perforated, the permeability increases significantly, why micro perforated impermeable films may act as permeable films).

Useful breathable films may be any film with high permeability. Most frequently, polyurethane films are used, but any permeable film may be used including silicone films and membranes.

In one embodiment, the moist vapour permeable layer (224) is a breathable film. In one embodiment of the first aspect of the present invention, the moist vapour permeable layer (224) is a silicone film.

In one embodiment, the outer radially extending layer (230) has a permeability of 0 to 200 g/m2/24h. In one embodiment the outer radially extending layer has a permeability of 0 to 100 g/m2/24h. In one embodiment the outer radially extending layer has a permeability of 0 to 50 g/m2/24h or 0 to 20 g/m2/24h. In one embodiment of the first aspect of the present invention, the outer radially extending layer (230) comprises a multilayered co-extruded film. Useful impermeable films may be any film with low permeability. An example are Nexcel^{®} MF283, which is a Coextruded five-layer EVA/EVA/PVDC/EVA/EVA film from Sealed Air (MVTR 5-15 g/m2/24h).

The wafer construction should be attached to the peri-stomal skin, either via a belt structure or via adhesive means. When using adhesives, as permeability of the inner layer is important, such adhesives should be applied in a relatively thin layer and should not reduce the permeability too much. Opposite it is also important, that the wafer does not unintentionally drop off.

In one embodiment the inner radially extending layer (220) comprises a skin adhesive (222) for attaching the wafer to the peri-stomal skin. Such skin adhesives could for example be acrylates, polyurethane and silicone adhesives. One example of such adhesives could be DOW CORNING MG-2410 Silicone Adhesive.

To minimize the impact of the adhesive on permeability, one could coat the skin contacting adhesive in a pattern.

In one embodiment, the skin adhesive (222) is pattern coated on the inner radially extending layer (220). In one embodiment, the skin adhesive (222) is pattern coated covering from 25 to 75% of the area of the inner radially extending layer (220).

As described earlier, one purpose of the present disclosure is to allow the peri-stomal skin to perspire so skin adhesion remains sufficiently high. This is today not solved by prior art - in particular in the stoma-adjacent-area.

In the present disclosure, as the inner radially extending layer (220) is not full-welded onto the outer radial layer (230), there is formed a radial space (270) between the two radially extending layers. This space (270) is in fluid communication with the atmosphere, allowing the perspired moisture to escape from the wafer construction. Consequently, a wafer construction according to the present disclosure, allows the peri-stomal skin to stay dry (and healthy) opposite to wafer constructions that absorb moisture, for example via hydrocolloid adhesives. On these prior art wafers, even though they do absorb the moisture, the skin tends to be wetted by the absorbed moisture, which again damaged the skin and may lead to wounding and wound infection.

In the present disclosure, it may be an advantage to somehow facilitate the separation of the inner radially extending layer from the outer radially extending layer, by a separation means, such as foams and non-wovens.

In one embodiment of the first aspect of the present invention, the at least one radial space (270) comprises separations means (271); said separation means facilitating the separation of the inner radially extending layer (220) from the outer radially extending layer (230) in the radial space (270).

In one embodiment, the separations means (271) is a non-woven layer. In one embodiment, the separations means is an open cell foam structure.

In one embodiment, the separations means (271) is attached to the inner surface (238) of the outer layer (230). In one embodiment, the separations means is attached to the outer surface (226) of the inner layer (220).

In one embodiment, the separations means (271) comprises moisture-transporting capabilities.

In order for the wafer to be integral, the outer layer must be attached to the inner layer - at least when the system is fully applied to the peri-stomal skin. If not, the outer layer (and the collecting bag would drop off). Therefore, the wafer comprises attachment point(s) (260), attaching the outer and inner layers to each other, at least during use. These attachment point(s) (260) may be separate discrete points or one large structure or pattern.

In one embodiment, the wafer construction comprises at least one attachment point (260) for attaching the inner layer (220) to the outer layer (230). The attachment point(s) may be created via glue, adhesive, welding or the like. In one embodiment, the wafer construction comprises at least two attachment points (260) for attaching the inner layer (220) to the outer layer (230). In one embodiment, the attachment point(s) is located both in the central stoma-adjacent-area (240) and in the encircling area (250).

In one embodiment, the substantially moist vapour impermeable film (232) of the outer radially extending layer (230) is solely located in the central stoma-adjacent-area (240).

If the radial space (270) comprises separation means (271), in principle the separation means could be attached to both the outer layer (230) and the inner layer (220). In such an example, the outer and inner layers could be attached to each other via one large attachment point.

In one embodiment, the separations means (271) is attached to the inner surface (238) of the outer layer (230) and to the outer surface (226) of the inner layer (220).

The portion of the wafer construction intended to be right next to and in some cases contact the ostomy is the inner part of the central stoma-adjacent-area (240). This portion is called the stoma-contacting-portion (245). The stoma-contacting-portion may, depending on the level of contact to the stoma and the sensitivity of the ostomy, be soft and gentle towards the stoma. Such softness may be made via a gel-matrix or via some mouldable wax. The stoma-contacting-portion should not be sharp as it may damage the ostomy.

In one embodiment, the central stoma-adjacent-area (240) comprises a stoma-contacting-portion (245), said stoma-contacting-portion being adapted to contact the ostomy. In one embodiment, the stoma-contacting-portion (245) comprises a gel. In one embodiment of the first aspect of the present invention, the stoma-contacting-portion (245) comprises an adhesive gel. In one embodiment, the gel in the stoma-contacting-portion (245) is of a shore A hardness of less than 16, less than 12 or less than 8. In one embodiment, the thickness of the wafer in the stoma-contacting-portion (245) is at least 1 mm, at least 1.5 mm or at least 2 mm.

The second aspect of the present disclosure relates to a collecting system (100) application on the peri-stomal skin, said system comprising:
a. a wafer construction (200) according to the first aspect of the present disclosure; and
b. a collecting reservoir (300) for collecting the stomal output.

The collecting reservoir (300) in the second aspect of the present disclosure may be integrally formed with the wafer (200) similar to typical 1-piece systems or it may be detachably attached to the wafer as known from 2-piece systems.

When the system is integrally formed, the bag element (234) for engaging the wafer (200) to the collecting reservoir (300) is most radially outer positioned where the bag is attached to the wafer for example via welding, glue or adhesion.

When the collecting reservoir (300) is attached to the wafer (200), the bag element (234) is the coupling or adhesive arrangement for detachably attaching the collecting reservoir (300) to the wafer (200).

In one embodiment, the collecting system (100) comprises a skin adhesive (400) for supporting the attachment of the system towards the skin.

The skin adhesive is intended to be positioned between the skin contacting surface (221) of the wafer and the peri-stoma skin, supporting the adhesion of the system (100) to the peri-stomal skin. The skin adhesive (400) may be a liquid solvent based adhesive, an uncured in-situ curable adhesive or a layer of double-sided adhesive.

In one embodiment, the skin adhesive (400) comprises a solvent (410) for solubilizing the adhesive. In one embodiment, the solvent (410) is an oligo-methyl-di-siloxane chemical. In one embodiment of the second aspect of the present invention, the solvent (410) is hexamethyl-disiloxane.

In one embodiment, the skin adhesive (400) is a silicone adhesive. Examples of such silicone skin adhesives include Advabond from Advancis Medical and MG-2410 Silicone Skin Adhesive from Dow Corning.

In one embodiment, the collecting system (100) comprises a soluble adhesive (400) for application on the skin prior to application of the wafer.

In the stoma-contacting-portion (245), moist perspiration may be reduced if the inner (220) and outer layer (230) are joint via a ring-shaped attachment point (260a). In one embodiment, the ring-shaped attachment point (260a) is formed as an impermeable ring. Therefore, the stoma-contacting area may have a relative small width. On the other hand, to provide broader cutting opportunities for the user, a relative large width of the stoma-contacting-portion (245) is needed. One way of meeting these opposite directed needs in two-piece systems are to provide the stoma-contacting-portion as a separate member for attachment when being used.

In one embodiment, the collecting system (100) comprises a separate gel-ring for sealing the system directly on outer diameter of the ostomy, said separate gel-ring be being attachable to a portion of the outer surface (236) of the outer radially extending layer (230).

The third aspect of the present disclosure relates to a method of providing a wafer construction (200) or collecting system (100) according to the first or second aspect of the present invention, said method comprising the steps of:
a. providing a wafer construction (200) according to the first aspect of the present invention; and
b. packing the wafer construction.

In one embodiment, the method of providing a wafer construction (200) comprises the steps of:
a. providing an inner permeable layer (220);
b. providing an outer impermeable layer (230);
c. attaching the inner permeable layer to the outer layer in at least one attachment point; and
d. applying an adhesive on the skin contacting surface of the inner permeable layer.

In one embodiment, the method of providing a collecting system (100) comprises the steps of:
a. providing a collecting system according to the second aspect of the present disclosure; and
b. providing a skin adhesive for application on the peri-stomal skin.

In one embodiment, the skin adhesive is a liquid solubilized adhesive.

It will be appreciated that any combination of features and elements of the above described aspects, and/or embodiments may be combined in any suitable manner.

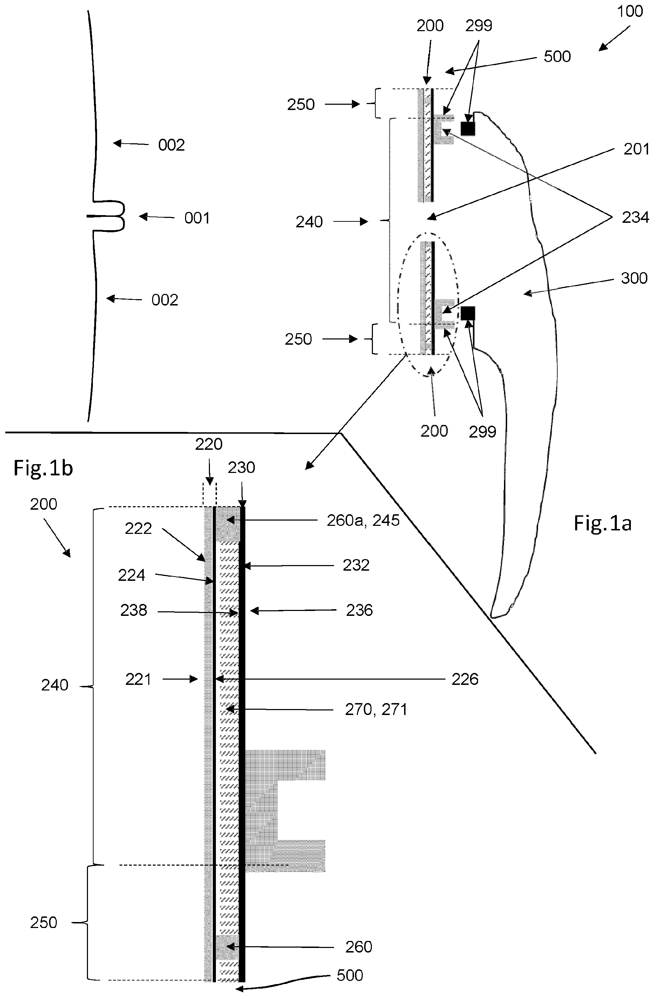

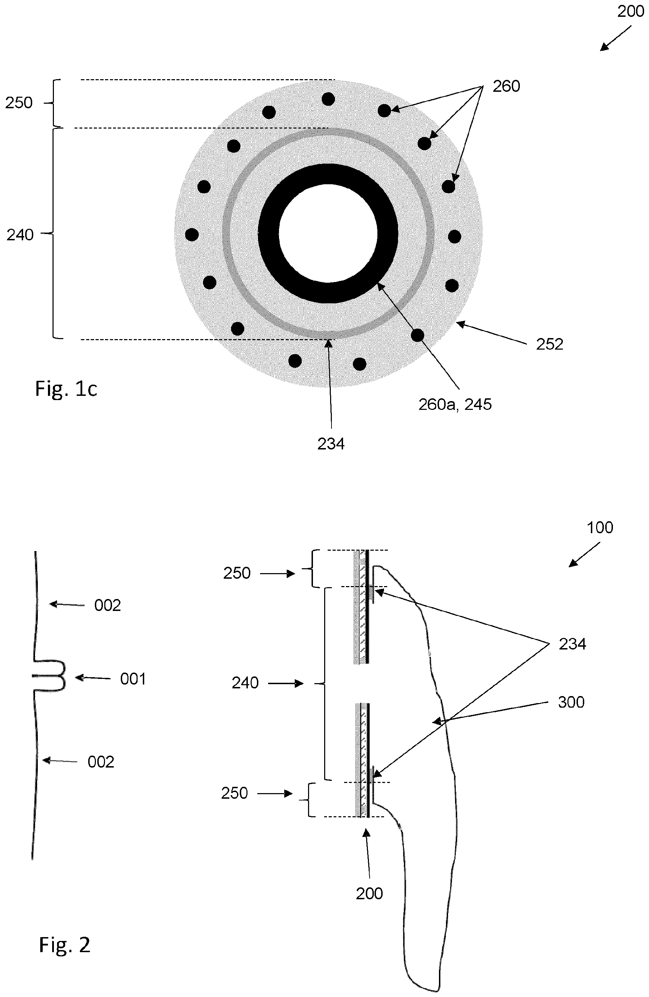

Figure 1 discloses a schematic cross-sectional illustration showing the main components of an example of a collecting system according to the present disclosure. Figure 1a illustrates the collecting system (100) in close proximity of an ostomy (001) and its peri-stomal skin (002). The system 100 comprises a wafer construction (200) and a collecting reservoir (300), which in this example is an ostomy bag. Figure 1b is a magnified cross-sectional, radial view of the wafer construction (200). Figure 1c is a schematic top view showing the main components of the wafer of this example. The illustrated example is a two-piece system, where the collecting reservoir (300) can be detachably attached to the wafer construction (200) via a coupling arrangement (299) located on both the wafer construction and on the collecting reservoir. Any coupling arrangement can be used, these are known in the prior art and will not be described in the present disclosure.

The wafer construction comprises a central through-going aperture (201) for receiving the ostomy (001). Furthermore, the wafer construction comprises an inner layer (220) and an outer layer (230), both intended to extend radially along the peri-stomal skin (002). The inner layer (220) comprises in this example a skin adhesive (222) on its skin contacting surface (221). In this example, the skin adhesive (222) is applied directly on a moist vapour permeable layer (224), which is a breathable film. The outer surface of the moist vapour permeable layer (224) constitutes the outer surface (226) of the inner layer (220).

The outer layer (230) of the wafer construction (200) comprises a moist vapour impermeable film (232), which outside surface in this example constitutes the outer surface (236) of the outer layer (230) and of the wafer (200). When the collecting reservoir (300) is attached to the wafer construction (200), the central area of the outer layer (230) of the wafer construction (200) participates in the wall structure of the collecting reservoir (300). In this example, the coupling arrangement (299) on the outer layer is the bag element (234).

The wafer construction (200) is defined to have radially two areas. A first central stoma-adjacent-area (240) and a second encircling area (250). The extent of the central stoma-adjacent-area (240) is defined by the position of the bag element (234) on the outer layer (230), in such a manner, that the central stoma-adjacent-area (240) is radially closer to the central through-going hole (201) than the bag element (234). The remaining portion of the wafer constitutes the encircling area (250).

As the outer and inner layers are not completely laminated or welded directly together, there is formed a radial space (270) between the two layers. The purpose of this space is to allow skin respiration during the use of the wafer and the collecting system. The space (270) must be in fluid communication with the atmosphere to allow optimal respiration. Moist vapour from skin respiration is transported through the permeable inner layer (220) and into the radial space (270). From the radial space (270) moisture is vented into the atmosphere (500).

However, as the inner and outer layer forms one wafer, they may be joined together by attachment point(s) (260). This may be via glue, adhesive, welding or other means for attachning the two layers together. The attachment point(s) (260) should be located in both the central stoma-adjacent-area (240) and in the encircling area (250). In this example, there are multiple attachment points (260) in the encircling area (250) and one ring-shaped attachment point (260a) in the central stoma-adjacent-area (240). The ring shaped attachment point (260a) is in one embodiment made of an impermeable material or welded together via an impermeable welded joint such that odours are not passed through the ring shaped attachment point. The attachment points may be seen on figure 1c (disclosing a schematic top view showing the main components of the wafer from figure 1, seen from the outer surface (236) of the outer layer (230)). The ring-shaped attachment point (260a) is the stoma-contacting-portion (245) of the wafer. In this example, the stoma-contacting-portion is made of a soft silicone gel with a width of 8 mm and a thickness of 2 mm. The stoma-contacting-portion (245) may be cut so the central through-going aperture (201) fits to the shape of the ostomy (001).

The wafer construction (200) may comprise separation means (271), which is disclosed in this example as a non-woven layer. The separation means is located between the inner layer (220) and outer layer (230). The separation means (271) may also act as a moisture transporter, but may also just be positioned to support the separation of the inner layer (220) and the outer layer (230) for improved venting of the space (270) to the atmosphere (500). The spacing layer of this example is disclosed on figure 1b.

Figure 2 illustrates a cross-sectional view of a 1-piece collecting system (100) similar to the 2-piece system illustrated in figure 1. The coupling arrangement (299) from figure 1 is replaced with a welding causing the wafer (200) to be non-detachably attached to the collecting reservoir (300). In this example, the welding is the bag element (234), which radially forms the outer boarder of the stoma-adjacent-area (240). The portion of the outer radially extending layer (230) that is located in the stoma-adjacent-area (240) participates in the formation of the collecting reservoir (300).

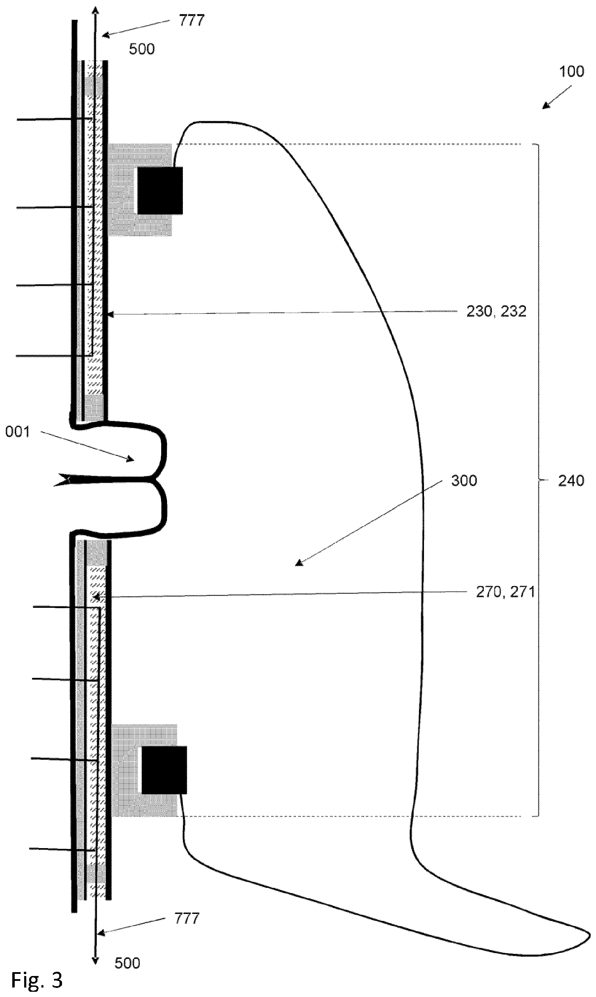

Figure 3 is a cross-sectional close-up of the wafer (200) and system (100) from example 1 applied onto the peri-stomal skin (002). The arrows (777) illustrate how peri-stomal skin perspiration can occur through the inner layer (220) to its outer surface (226) and into the radial space (270). As the radial space is in fluid communication with the atmosphere (500), the perspired moisture can escape the wafer construction and into the atmosphere. Note, that as opposed to prior art, the peri-stomal skin attached to the central stoma-adjacent-area of the wafer in the present disclosure is able to expel moisture to the atmosphere. As the outer layer (230) in the central stoma-adjacent-area (240) comprises an impermeable layer (232), mal-odour from the ostomy (001) and from the collecting reservoir (300) is not able to migrate to the atmosphere via the radial space (270). The spacing means (271) in the radial space (270) support the separation of the outer and inner layer and thereby improve the perspiration.

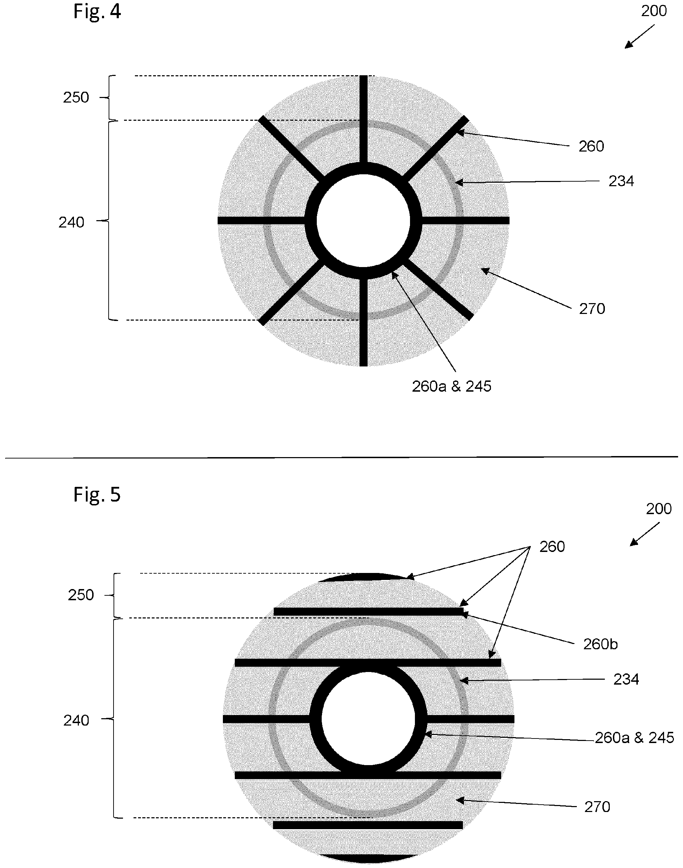

Figure 4 is a schematic top view showing the main components of another example of a wafer according to the first aspect of the present disclosure. The main difference from figure 1 (figure 4 is directly comparable to figure 1c), is that the attachment point (260) is one large attachment, shaped like a star around the central through-going aperture (201). In this example, the attachment point is a 2.5 mm thick adhesive gel. The central ring shape (260a) of the attachment point (260) is the stoma-contacting-portion (245) of the wafer. The stoma-contacting-portion may be cut to fit the central through-going aperture (201) to the shape of the ostomy (001).

It should be noted, that in this example, due to the star shaped structure of the attachment point (260), there are 8 radial spaces (270).

Figure 5 is a schematic top view showing the main components of another example of a wafer according to the first aspect of the present disclosure. The main difference from figure 1 and 4 (figure 5 is directly comparable to figure 1c and 4), is that the attachment points (260) are a central stomal-encircling ring (260a) and a plurality of horizontal lines (260b). The central ring shape (260a) of the attachment points (260) is the stoma-contacting-portion (245) of the wafer. It should be noted, that in this example, due to the horizontally lined orientation of the attachment points (260), there are a plurality of horizontally oriented radial spaces (270). This design may on some obese patients better fit to the natural skin folds and thereby increase patient comfort.

In principle, the outer layer (230) only needs to comprise a moist vapour impermeable film (232) in central stoma-adjacent-area (240). Outside - in the encircling area (250) of the wafer (200), the outer layer (230) could be a moist vapour permeable layer, such as a non-woven, a film or not exist at all. However if the outer layer extends throughout the encircling area and there exists attachment points in this area, these support the distribution of force of from the attachment of the collecting reservoir (300) via the wafer (200) to the peri-stomal skin.

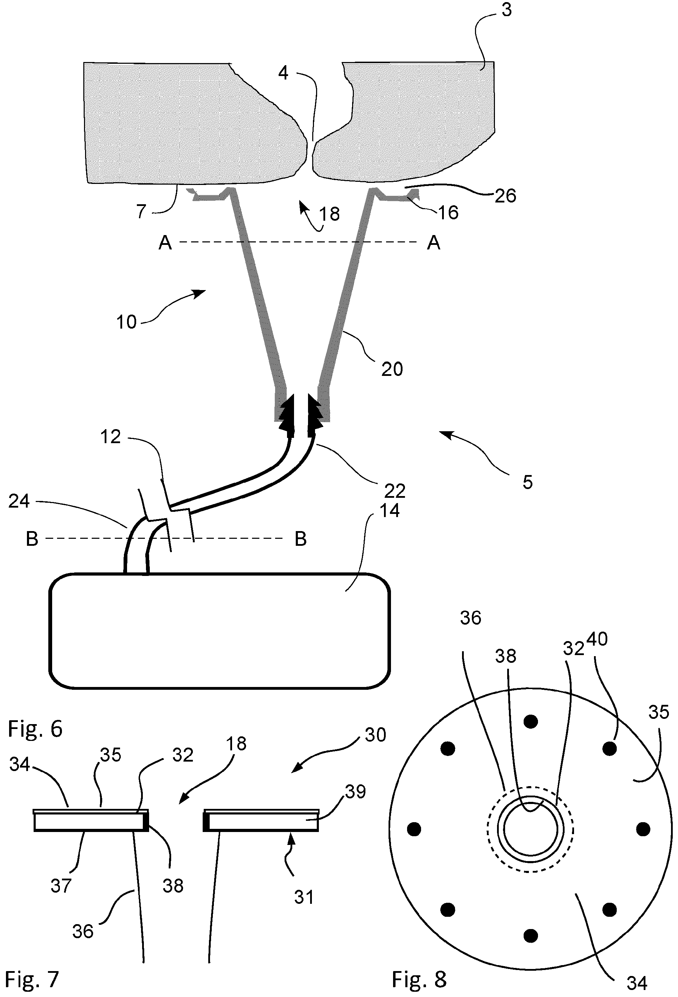

Figure 6 discloses a very schematic illustration showing the main components of the body waste collecting system of the invention in the form of a perianal faecal collecting system. It should be noted that in this current embodiment, the faecal collecting system is arranged to be used together only with an adhesive, however it should be clear to the person skilled in the art that the faecal collecting system could also used in combination with mechanical fastening systems.

The system 5 is arranged to be attached to the perianal skin 7 of a user 3 around the anal orifice 4 and collect faecal waste exiting the anus 4 of the user. The system 5 in general comprises an attachment member 10, a conduit or tubular section 12 and a collection bag 14. The attachment member is arranged to establish a fluid tight connection between the system and the user. The conduit is arranged to establish fluid communication between the attachment member and the collection bag. The collection bag is arranged to collect the faecal output. This type of system is described in more detail in WO 2018/050856 and WO2019/179586,

The attachment member 10 comprises an attachment flange 16 which is attached to the peri-anal skin of the user. The attachment member 10 of the current embodiment also comprises an opening 18 and an elongated portion 20. The conduit 12 has two ends, a first end 22 in fluid communication with the attachment member and a second end 24 in fluid communication with the collection bag.

In the current schematic embodiment, there are three separate elements, an attachment member 10, a conduit 12 and a collection bag 14. However, within the scope of the invention, these elements could be integrated together in different combinations. For example, an embodiment could be made which comprises a single integrated element. In another embodiment, the conduit and attachment member could be integrated into a single element, or the conduit and the collection bag could be integrated into a single element. Likewise, it should be mentioned that the conduit and the elongated portion of the attachment member fulfil similar functions.

For the sake of the current specification and for the understanding of the claims, the attachment member should be understood as the part of the system which establishes a fluid tight communication with the anus or stoma of the user, the conduit should be understood as that part of the system which establish fluid communication between the attachment member and the collection bag, and the collection bag is the part of the system which collects the faecal or urinary waste from the user.

In certain embodiments, the "conduit" could be very short such that the collection bag is attached almost directly to the attachment flange. More concretely, in certain embodiments, the conduit is less than 5cm long, less than 4cm long or less than 3cm long. In other embodiments, the conduit is longer allowing the collection bag to be placed further from the attachment flange. In certain embodiments, the conduit is greater than 10cm long, greater than 20cm long or greater than 30cm long.

In the embodiment shown in figure 6, it can therefore be understood that the portion of the system which is above the line AA is considered the attachment member, the portion which lies under the line BB is considered the collection bag and the portion which lies between the lines AA and BB is considered the conduit. Using this understanding, in one embodiment the conduit should be at least 30 cm long and less than 8 cm in diameter. When compared to prior art solutions, this embodiment is typically longer and/or thinner than any systems which are attached externally to the perianal or peristomal skin.

In the current embodiment shown in figure 6, the attachment flange 16 is attached to the perianal skin via an adhesive 26 applied between the attachment flange and the perianal skin. Many suitable forms of adhesive have been discussed in the prior art. We refer expressly to applicant's own prior art mentioned above. In another embodiment, instead of an adhesive, a mechanical system could be used with straps to press the attachment flange against the peri-anal skin. Adhesive and mechanical systems could also be combined if desired.

Figure 7 shows an embodiment of an attachment member 30 according to the invention. The attachment member comprises an attachment flange 31 and a tubular section 36. Here the attachment flange comprises a first inner annular portion 32 is arranged closest to the opening 18 and a second outer annular portion 34 is arranged farther away from the opening. The tubular section 36 is attached to the attachment flange 31 between the inner and outer annular portions 32,36. In this way, a cross section through one side of the attachment member will be in the shape of T. Since the tubular portion is attached to the attachment flange at a location away from the inside edge of the attachment flange, forces applied to the tubular section will be less likely to peal the attachment flange away from the skin of the user.

The embodiment of figure 7 and 8 is made up of two layers, an inner permeable layer 35 and an outer impermeable layer 37. The two layers are joined at the centre via an impermeable joint 38. The two layers are joined together around the periphery of the flange via attachment points 40. A air permeable material 39 is provided between the inner and outer layers.

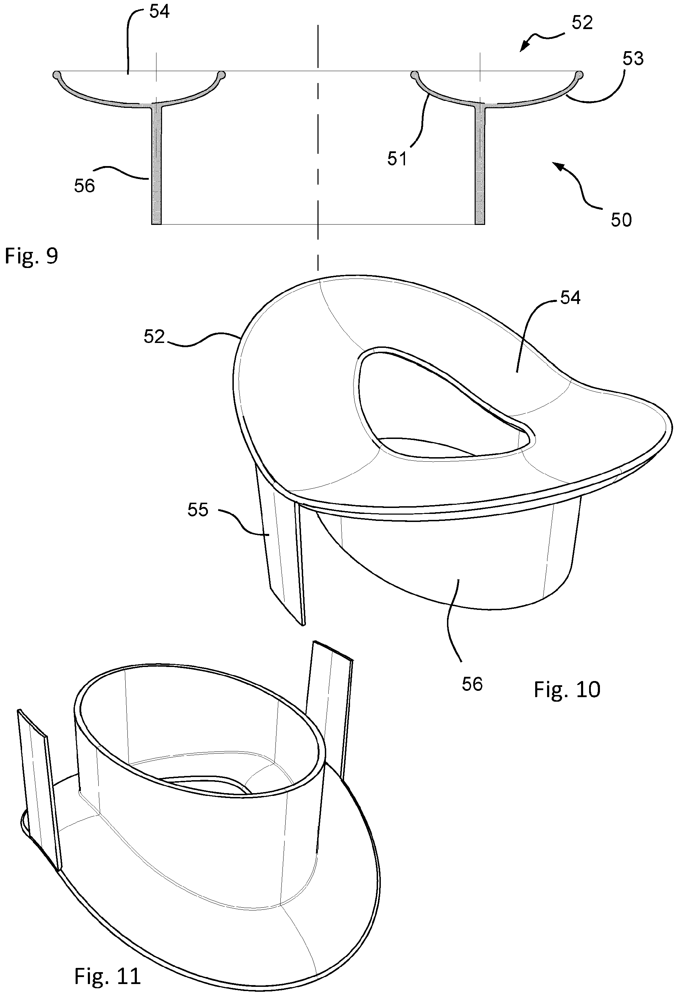

It should be noted that figure 6 shows a prior art type of attachment member where the tubular section 20 is attached at the inside of the attachment flange 16. This is different from the embodiments of figure 7 and 8, as well as the embodiments in figures 9-17.

Figures 9-11, show different views of another embodiment of an attachment member suitable for a body waste collecting system according to the second invention. In this case, the attachment member 50 comprises an attachment flange 52 comprising an annular groove 54 filled with an adhesive (not shown). As can be seen from the cross section of figure 9, the tubular section 56 of the attachment member is attached inwardly from the edge of the opening 58. In this way, a portion 51 of the attachment flange is arranged inwardly of the tubular section and a portion 53 of the attachment flange is arranged outwardly of the tubular section. Straps 55 are arranged on either side of the attachment flange to help with removing the attachment flange from the user.

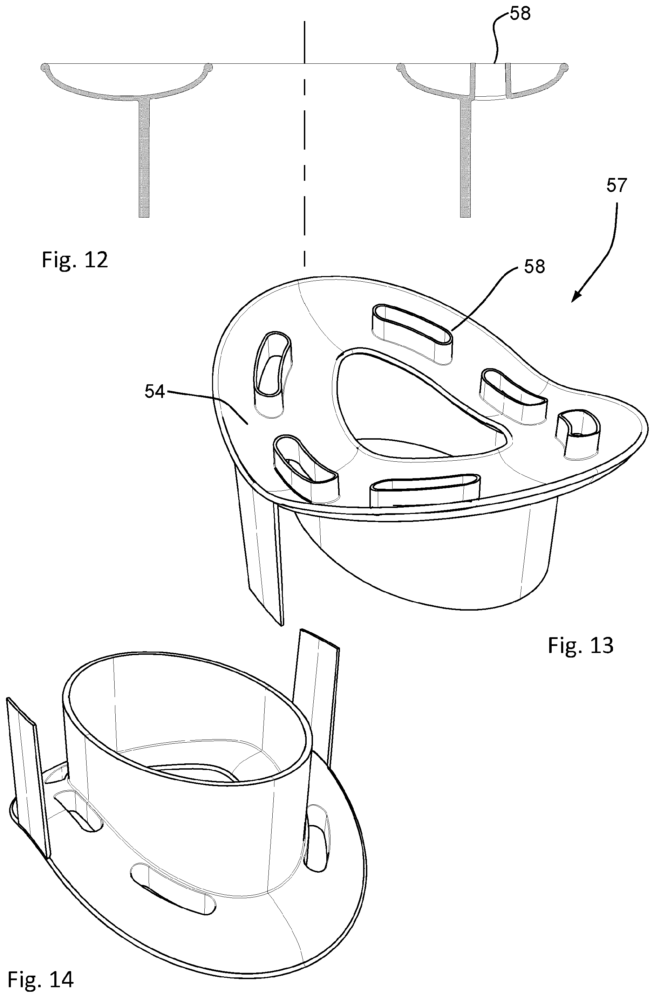

Figures 12-14 show different views of another embodiment 57 of an attachment member suitable for a body waste collecting system according to the invention. This embodiment is almost identical to the embodiment of figures 9-11 and as such it will not be described in detail here. However, the main difference is that chimneys 58 are provided in the annular channel extend upwardly from the bottom surface of the annular groove 54. These chimneys extend upwardly through the adhesive which is filled in the groove, thereby allowing extra breathing of the skin of the user through the chimneys.

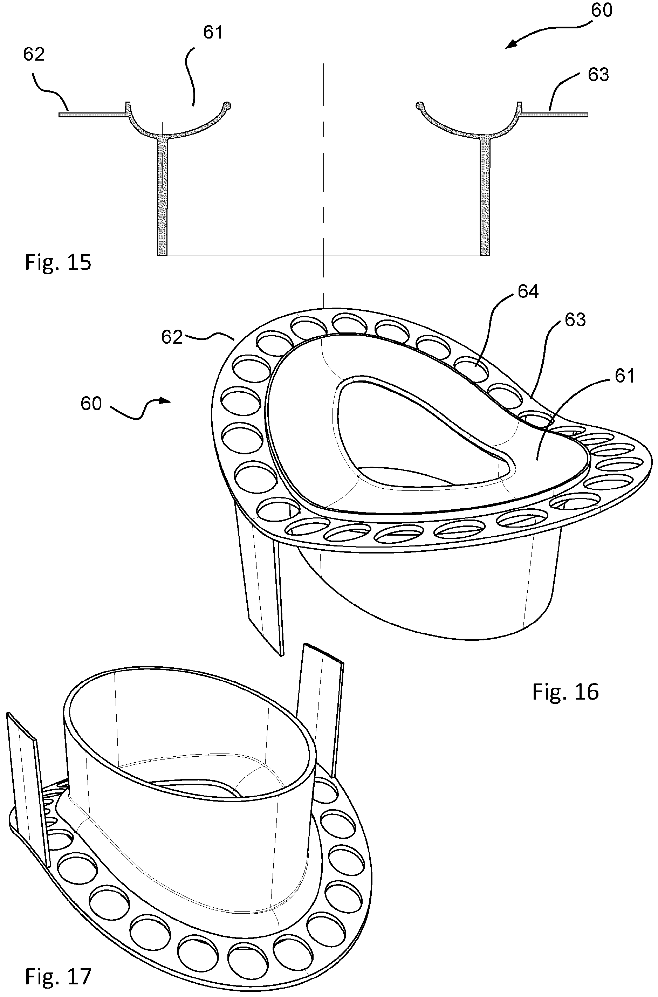

Figures 15-17 show different views of another embodiment 60 of an attachment member. This embodiment is also very similar to the previous two embodiments and will not be described in great detail. However the main difference is that the annular groove 61 has a smaller width and on the outside of the annular groove, an outer annular flange 62 is provided with through going openings 64. In one embodiment, the skin facing surface 63 of the outer annular flange 62 is provided with an adhesive (not shown). The outer annular flange will therefore provide a form of attachment to the skin of the user, while still providing a very large amount of permeability. In another embodiment, a thin permeable film (not shown) could be applied on top of the outer annular flange 62 and an adhesive could be applied to the surface of the thin permeable film. This will provide a greater strength while also providing a permeable connection to the user's skin.

As can be seen from the cross section figures 9, 12 and 15, the tubular section is attached to the attachment flange in all the embodiments on the bottom surface and in the centre of the annular groove. This will also allow the adhesive to absorb more of the deformations before the material of the flange itself starts to peel away from the skin of the user.

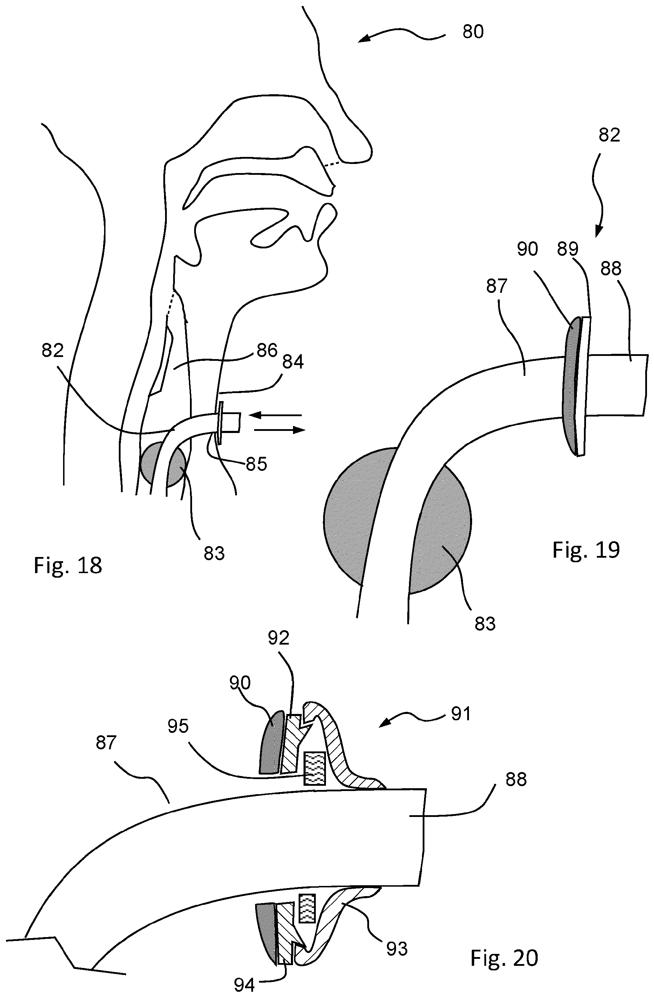

Figure 18 shows a side view of a user 80 with a tracheostomy tube 82 attached to the skin 84 of the neck of the user. The tracheostomy tube 82 is inserted through a tracheostomy 85 and into the trachea 86 of the user. An expandable balloon 83 is provided in this embodiment to seal the trachea of the user.

Figure 19 shows a more detailed view of a tracheostomy tube assembly suitable for use in the application shown in figure 18. The tracheostomy tube assembly 82 comprises an internal tubular portion 87 and an external tubular portion 88. The internal tubular portion is inside the user during normal use while the external portion extends out of the user during normal use. An attachment flange 89 is provided extending perpendicularly outwardly from the external tubular portion. A skin friendly adhesive 90 is provided on a skin facing surface of the attachment flange 89.

When the tracheostomy tube is to be inserted into the user, the adhesive could already by applied to the attachment flange, or the adhesive could be applied to the attachment flange just prior to inserting the tube into the user.

Figure 20 shows another embodiment 91 of a tracheostomy tube assembly. In this assembly, the attachment flange 92 is not integrally connected to the external tubular portion 88 of the tracheostomy tube, but is detachably connected to the tube via a fastening member 93. In this embodiment, the attachment flange 92 is provided with a skin friendly adhesive 90 on the skin facing side of the attachment flange 92. The attachment flange is in this embodiment formed as an annular flange which extends around the tracheostomy. The attachment flange can be attached to the user via the adhesive and left in place for a prolonged period of time. The tube can then be inserted into the trachea of the user, and the fastening member 93 is engaged with engaging elements 94 on the attachment flange. In one embodiment, the fastening member could be formed as an annular elastic coupling element which detachably snaps onto a ring formed engaging element 94 on the attachment member.

When it is desired to replace the tube, the fastening member can be detached and the tube removed. **In** one embodiment, the fastening member can be integrally or at least semi permanently connected to the external tubular portion, for example via a press fit connection as shown in the current embodiment. **In** another embodiment it could be detachably connected to the external tubular portion, for example via another form of snap coupling (not shown).

An annular absorbing element 95 could be arranged in the gap between the attachment member and the fastening member to absorb fluid exiting the user via the tracheostomy. By opening the fastening member at regular intervals, the absorbing element could be removed. It is also possible to apply suction to the volume between the attachment member and the fastening member to removed fluids trapped in between.

## Claims

1. A body waste collecting system comprising
a. an attachment member arranged to be attached to the user, said attachment member comprising an opening which is arranged to be in fluid communication with the anus of the user and an attachment flange which is arranged to encircle said opening and which is arranged to be attached to the perianal skin of the user such that a fluid tight seal is established between the attachment flange and the perianal skin of the user around the anus, said attachment flange being provided with a skin friendly adhesive on a skin contacting surface of said attachment flange, and
b. a collection bag connected to the attachment member, said collection bag being suitable for collecting the body waste output, wherein
c. the attachment flange is provided with an inner flange portion arranged closest to the opening in the attachment member and an outer flange portion arranged further away from the opening in the attachment member than the inner flange portion and a tubular section extending away from the attachment flange, said tubular section being in fluid communication with the opening in the attachment flange and being attached to the attachment flange at a location between the inner and outer flange portions.

2. A body waste collecting system according to claim 1, **characterized in that** the attachment flange comprises an annular groove between the inner and outer flange portions.

3. A body waste collecting system according to claim 2, **characterized in that** the annular groove is filled or is able to be filled with an adhesive.

4. A body waste collecting system according to claim 3, **characterized in that** the adhesive in the groove has an elongation at break of from 150 to 4000 %.

5. A body waste collecting system according to any one of claims 2-4, **characterized in that** the attachment flange comprises an annular flange portion on the inside and/or the outside of the annular groove, said annular flange(s) being provided with a skin friendly adhesive.

6. A body waste collecting system according to any one of claims 1 to 5, **characterized in that** the annular flange portion(s) is/are provided with through going openings to allow skin arranged underneath the annular flange portion(s) to breathe.

7. A body waste collecting system according to claim 6, **characterized in that** the through going openings in the annular flange portions are covered by a thin permeable film which is covered by a skin friendly adhesive.

8. A body waste collecting system according to any one of claims 2-7, **characterized in that** the attachment member comprises elongated chimneys circularly arrayed in the annular groove and extending perpendicularly to the plane of the groove, said chimneys connecting the user's skin to the outer surface of the attachment flange.

9. A body waste collecting system according to claim 8, **characterized in that** the chimneys are surrounded by adhesive.

10. A body waste collecting system according to any one of claim 1 to 9, **characterized in that** the attachment flange of the attachment member is arranged as a two layer structure comprising an inner permeable film and an outer impermeable film separated by an air permeable gap.

## Patentansprüche

1. Körperausscheidungsauffangsystem, umfassend:
a. ein Befestigungselement, das dazu angeordnet ist, an dem Benutzer befestigt zu sein, das Befestigungselement umfassend eine Öffnung, die dazu angeordnet ist, in fluidischer Kommunikation mit dem Anus des Benutzers zu sein, und einen Befestigungsflansch, der dazu angeordnet ist, die Öffnung zu umschließen und der dazu angeordnet ist, an der perianalen Haut des Benutzers derart befestigt zu sein, dass zwischen dem Befestigungsflansch und der perianalen Haut des Benutzers um den Anus herum eine fluiddichte Abdichtung hergestellt wird, wobei der Befestigungsflansch mit einem hautfreundlichen Kleber auf einer Hautkontaktfläche des Befestigungsflansches versehen ist, und
b. einen Auffangbeutel, der mit dem Befestigungselement verbunden ist, wobei der Auffangbeutel zum Auffangen des Körperausscheidungsausstoßes geeignet ist, wobei
c. der Befestigungsflansch mit einem inneren Flanschbereich, der am nächsten zu der Öffnung in dem Befestigungselement angeordnet ist, und einem äußeren Flanschbereich, der weiter weg von der Öffnung in dem Befestigungselement als der innere Flanschbereich angeordnet ist, und einem schlauchförmigen Abschnitt, der sich von dem Befestigungsflansch weg erstreckt, versehen ist, wobei der schlauchförmige Abschnitt in fluidischer Kommunikation mit der Öffnung in dem Befestigungsflansch ist und an dem Befestigungsflansch an einer Stelle zwischen dem inneren und dem äußeren Flanschbereich befestigt ist.

2. Körperausscheidungsauffangsystem nach Anspruch 1, **gekennzeichnet dadurch, dass** der Befestigungsflansch eine ringförmige Rille zwischen dem inneren und dem äußeren Flanschbereich umfasst.

3. Körperausscheidungsauffangsystem nach Anspruch 2, **gekennzeichnet dadurch, dass** die ringförmige Rille mit einem Kleber gefüllt ist oder gefüllt werden kann.

4. Körperausscheidungsauffangsystem nach Anspruch 3, **gekennzeichnet dadurch, dass** der Kleber in der Rille eine Bruchdehnung von 150 bis 4000 % aufweist.

5. Körperausscheidungsauffangsystem nach einem der Ansprüche 2 bis 4, **gekennzeichnet dadurch, dass** der Befestigungsflansch einen ringförmigen Flanschbereich auf der Innenseite und/oder der Außenseite der ringförmigen Rille umfasst, wobei der/die ringförmige(n) Flansch(e) mit einem hautfreundlichen Kleber versehen ist/sind.

6. Körperausscheidungsauffangsystem nach einem der Ansprüche 1 bis 5, **gekennzeichnet dadurch, dass** der/die ringförmige(n) Flanschbereich(e) mit durchgehenden Öffnungen versehen ist/sind, um zu erlauben, dass Haut, die unter dem/den ringförmige(n) Flanschbereich(en) angeordnet ist, atmen kann.

7. Körperausscheidungsauffangsystem nach Anspruch 6, **gekennzeichnet dadurch, dass** die durchgehenden Öffnungen in den ringförmigen Flanschbereichen mit einem dünnen durchlässigen Film bedeckt sind, der mit einem hautfreundlichen Kleber bedeckt ist.

8. Körperausscheidungsauffangsystem nach einem der Ansprüche 2 bis 7, **gekennzeichnet dadurch, dass** das Befestigungselement längliche Kamine umfasst, die kreisförmig in der ringförmigen Rille aufgestellt sind und sich senkrecht zu der Ebene der Rille erstrecken, wobei die Kamine die Haut des Benutzers mit der äußeren Fläche des Befestigungsflansches verbinden.

9. Körperausscheidungsauffangsystem nach Anspruch 8, **gekennzeichnet dadurch, dass** die Kamine von Kleber umgeben sind.

10. Körperausscheidungsauffangsystem nach einem der Ansprüche 1 bis 9, **gekennzeichnet dadurch, dass** der Befestigungsflansch des Befestigungselements als eine zweischichtige Struktur angeordnet ist, die einen inneren durchlässigen Film und einen äußeren undurchlässigen Film umfasst, die durch einen luftdurchlässigen Spalt getrennt sind.

## Revendications

1. Système de collecte de déchets corporels comprenant
a. un élément de fixation agencé pour être fixé à l'utilisateur, ledit élément de fixation comprenant une ouverture qui est agencée pour être en communication fluidique avec l'anus de l'utilisateur et une bride de fixation qui est agencée de sorte à encercler ladite ouverture et qui est agencée de sorte à être fixée à la peau périanale de l'utilisateur de telle sorte qu'un joint étanche aux fluides est établi entre la bride de fixation et la peau périanale de l'utilisateur autour de l'anus, ladite bride de fixation étant pourvue d'un adhésif respectueux de la peau sur une surface en contact avec la peau de ladite bride de fixation, et
b. un sac de collecte relié à l'élément de fixation, ledit sac de collecte étant approprié pour collecter les déchets corporels évacués, dans lequel
c. la bride de fixation est pourvue d'une partie de bride interne agencée au plus près de l'ouverture dans l'élément de fixation et d'une partie de bride externe agencée plus loin de l'ouverture dans l'élément de fixation que la partie de bride interne et d'une section tubulaire s'étendant à partir de la bride de fixation, ladite section tubulaire étant en communication fluidique avec l'ouverture dans la bride de fixation et étant fixée à la bride de fixation au niveau d'un emplacement entre les parties de bride interne et externe.

2. Système de collecte de déchets corporels selon la revendication 1, **caractérisé en ce que** la bride de fixation comprend une rainure annulaire entre les parties de bride interne et externe.

3. Système de collecte de déchets corporels selon la revendication 2, **caractérisé en ce que** la rainure annulaire est remplie ou est susceptible d'être remplie d'un adhésif.

4. Système de collecte de déchets corporels selon la revendication 3, **caractérisé en ce que** l'adhésif dans la rainure a un allongement à la rupture de 150 à 4000 %.

5. Système de collecte de déchets corporels selon l'une quelconque des revendications 2 à 4, **caractérisé en ce que** la bride de fixation comprend une partie de bride annulaire sur l'intérieur et/ou l'extérieur de la rainure annulaire, ladite/lesdites bride(s) étant pourvue(s) d'un adhésif respectueux de la peau.

6. Système de collecte de déchets corporels selon l'une quelconque des revendications 1 à 5, **caractérisé en ce que** la/les partie(s) de bride annulaire est/sont pourvue(s) d'ouvertures traversantes pour permettre à la peau agencée sous la/les partie(s) de bride annulaire de respirer.

7. Système de collecte de déchets corporels selon la revendication 6, **caractérisé en ce que** les ouvertures traversantes dans les parties de bride annulaire sont recouvertes par un film perméable mince qui est recouvert par un adhésif respectueux de la peau.

8. Système de collecte de déchets corporels selon l'une quelconque des revendications 2 à 7, **caractérisé en ce que** l'élément de fixation comprend des cheminées allongées disposées de manière circulaire dans la rainure annulaire et s'étendant perpendiculairement au plan de la rainure, lesdites cheminées reliant la peau de l'utilisateur à la surface externe de la bride de fixation.

9. Système de collecte de déchets corporels selon la revendication 8, **caractérisé en ce que** les cheminées sont entourées par de l'adhésif.

10. Système de collecte de déchets corporels selon l'une quelconque des revendications 1 à 9, **caractérisé en ce que** la bride de fixation de l'élément de fixation est agencée sous forme d'une structure à deux couches comprenant un film perméable interne et un film imperméable externe séparés par un espace perméable à l'air.
